(19) Europäisches Patentamt
European Patent Office
Office européen des brevets

(11) Veröffentlichungsnummer: **0 131 837**
**A1**

(12) # EUROPÄISCHE PATENTANMELDUNG

(21) Anmeldenummer: **84107668.0**

(22) Anmeldetag: **03.07.84**

(51) Int. Cl.⁴: **C 07 C 13/28**
C 07 C 25/18, C 07 C 121/00
C 07 D 319/06, C 09 K 19/30
C 09 K 19/34

(30) Priorität: **16.07.83 DE 3325727**

(43) Veröffentlichungstag der Anmeldung:
**23.01.85 Patentblatt 85/4**

(84) Benannte Vertragsstaaten:
**CH DE GB LI**

(71) Anmelder: **Merck Patent Gesellschaft mit beschränkter Haftung**
**Frankfurter Strasse 250**
**D-6100 Darmstadt(DE)**

(72) Erfinder: **Fuss, Peter, Dr.**
**Im Seegraben 5**
**D-6109 Mühltal-Traisa(DE)**

(72) Erfinder: **Eidenschink, Rudolf, Dr.**
**Kornblumenstrasse 1**
**D-6115 Münster(DE)**

(54) **Flüssigkristalline Verbindungen.**

(57) Die Anmeldung betrifft Verbindungen der Formel I

$R^1 - A^1 - X - (A^2)_n - Y - A^3 - R^2$

worin
$A^1$, $A^2$ und $A^3$ jeweils unabhängig voneinander einen Ring der Formel (1) oder (2),

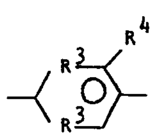

(1)

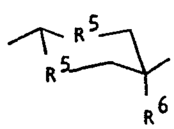

(2)

n 1 oder 2,
X und Y jeweils unabhängig voneinander –O–, –S–, $-CH_2-$, einer davon auch $-CHR^7-$, –CH=CH–Q, $-OCO-CH_2-$, $-COO-CH_2-$ oder $-O-CH_2-O-$,
$R^1$ und $R^2$ Alkyl, Alkoxy oder Alkanoyloxy mit jeweils bis zu 8 C-Atomen, einer davon auch H, CN, F, Cl oder Br,
Q O, S oder $CH_2$,
$R^3$ CH oder N,
$R^4$ H, CN, F, Cl, Br, $CH_3$ oder $OCH_3$,
$R^5$ $CH_2$ oder O,
$R^6$ H, CN, F, Cl oder Br und
$R^7$ CN, F, Cl oder Br bedeutet.

EP 0 131 837 A1

Croydon Printing Company Ltd

Merck Patent Gesellschaft
mit beschränkter Haftung
D a r m s t a d t

# Flüssigkristalline Verbindungen

Die Erfindung betrifft neue flüssigkristalline Verbindungen der Formel I

$$R^1 - A^1 - X - [A^2]_n - Y - A^3 - R^2$$

worin

$A^1$, $A^2$ und $A^3$ jeweils unabhängig voneinander einen Ring der Formel (1) oder (2),

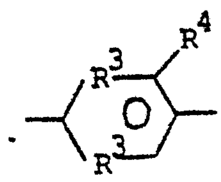
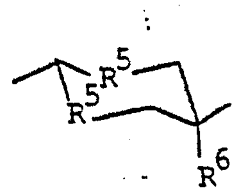

(1) (2)

| | |
|---|---|
| n | 1 oder 2, |
| X und Y | jeweils unabhängig voneinander $-O-$, $-S-$, $-CH_2-$, einer davon auch $-CHR^7-$, $-CH{=}CH-Q$, $-OCO-CH_2-$, $-COO-CH_2-$ oder $-Q-CH_2-Q-$, |

| | |
|---|---|
| $R^1$ und $R^2$ | Alkyl, Alkoxy oder Alkanoyloxy mit jeweils bis zu 8 C-Atomen, einer davon auch H, CN, F, Cl oder Br, |
| Q | O, S oder $CH_2$, |
| $R^3$ | CH oder N, |
| $R^4$ | H, CN, F, Cl, Br, $CH_3$ oder $OCH_3$, |
| $R^5$ | $CH_2$ oder O, |
| $R^6$ | H, CN, F, Cl oder Br und |
| $R^7$ | CN, F, Cl oder Br bedeutet, |

die zur Verwendung als Komponenten flüssigkristalliner Dielektrika bestimmt sind, und neue flüssigkristalline Dielektrika mit mindestens einer Verbindung der Formel I, die sich zur Verwendung in elektrooptischen Anzeigevorrichtungen eignen.

Es ist bereits eine Anzahl von flüssigkristallinen Verbindungen bekannt, die den an Dielektrika für elektronische Bauelemente gestellten Stabilitätsanforderungen teilweise genügen. Die wichtigsten in Frage kommenden Verbindungen lassen sich durch die Formel II charakterisieren:

$$R^8 - A - G - E - R^9$$

worin A und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | $-COO-C_6H_4-COO-$ |
| | -CH=CH-CO-O- | |
| | oder eine C-C-Einfachbindung, | |

Y Halogen, vorzugsweise Chlor, oder -CN, und $R^8$ und $R^9$ Alkyl, Alkoxy, Alkanoxyloxy oder Alkoxycarbonyloxy mit bis zu 18, vorzugsweise bis zu 8 Kohlenstoffatomen, oder einer dieser Reste auch -CN, -NC, $-NO_2$, $-CF_3$, F, Cl oder Br bedeuten. Bei den meisten dieser Verbindungen sind $R^8$ und $R^9$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle können in an sich üblicher Weise nach Standardverfahren hergestellt werden. Bei allen bisher bekannten Flüssigkristallsubstanzen mit mehreren carbo- oder heterocyclischen Ringen sind diese über eine gerade Zahl von Atomen (0, 2, 4) in Brückengliedern G verknüpft.

In den genannten Verbindungsklassen wie auch in anderen bekannten Reihen von Verbindungen mit flüssigkristalliner Mesophase, beispielsweise ausgewählt aus den Klassen der Azoxybenzole, Benzylidenaniline, Biphenyle, Terphenyle, Phenyl- oder Cyclohexylbenzoate, Cyclohexancarbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbiphenyle, Phenyl- oder Cyclohexylpyrimidine, Phenyl- oder Cyclohexyldioxane, ggf. halogenierte Stilbene, Benzylphenylether, Tolane und substitutierten Zimtsäuren, gibt es keine Einzelverbindungen, die in den geforderten Temperaturbereichen eine flüssigkristalline nematische Mesophase ausbilden. Es werden daher in der Regel Mischungen von zwei oder

mehreren Verbindungen hergestellt, um als flüssigkristalline Dielektrika verwendbare Substanzen zu erhalten. Hierzu mischt man gewöhnlich mindestens eine Verbindung mit niedrigem Schmelz- und Klärpunkt mit einer anderen mit deutlich höherem Schmelz- und Klärpunkt. Hierbei wird normalerweise ein Gemisch erhalten, dessen Schmelzpunkt unter dem der niedriger schmelzenden Komponente liegt, während der Klärpunkt zwischen den Klärpunkten der Komponenten liegt. Trotzdem bereitet die Herstellung optimaler Dielektrika immer wieder Schwierigkeiten, da die Komponenten mit den hohen Schmelz- und Klärpunkten den Gemischen häufig auch eine hohe Viskosität verleihen. Dadurch werden die Schaltzeiten der damit hergestellten elektrooptischen Anzeigeelemente in unerwünschter Weise verlängert. Ferner treten oft Probleme dadurch auf, daß die Löslichkeit der verschiedenen Komponenten ineinander, insbesondere bei Raumtemperatur oder tieferen Temperaturen nur sehr begrenzt ist.

Der Erfindung lag die Aufgabe zugrunde, neue stabile flüssigkristalline oder mesogene Verbindungen aufzufinden, die als Komponenten flüssigkristalliner Dielektrika geeignet sind. Diese Aufgabe wurde durch die Bereitstellung der Verbindungen der Formel I gelöst.

Es wurde gefunden, daß die Verbindungen der Formel I, deren Brückenglieder die carbo- oder/und heterocyclischen Ringe über eine ungerade Zahl von Atomen verknüpfen, vorzüglich als Komponenten flüssigkristalliner Dielektrika geeignet sind.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I eignen sich ferner als Zwischenprodukte zur Herstellung anderer Substanzen, die sich als Bestandteile flüssigkritalliner Dielektrika verwenden lassen.

Durch geeignete Auswahl der Substituenten $R^1$ und $R^2$ sowie der Art der Ringe $A^1$, $A^2$ und $A^3$ sowie der Brückenglieder X und Y lassen sich mit den Verbindungen der Formel I sowohl Dielektrika mit positiver dielektrischer Anisotropie zur Verwendung in Anzeigeelementen auf der Basis der verdrillten nematischen Zelle oder des cholesterisch-nematischen Phasenübergangs herstellen, es können aber auch Dielektrika mit von Null nur wenig verschiedener oder auch mit negativer dielektrischer Anisotropie hergestellt werden, die in Anzeigeelementen auf der Basis der dynamischen Streuung oder der Deformation aufgerichteter Phasen (DAP-Effekt) verwendet werden.

Die Verbindungen der Formel I können wie ähnliche Verbindungen als Komponenten flüssigkristalliner Dielektrika verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I und ihre Verwendung als Komponenten flüssigkristalliner Dielektrika. Gegenstand der Erfindung sind weiterhin flüssigkristalline Dielektrika mit einem Gehalt an Verbindungen der Formel I sowie elektrooptische Anzeigeelemente auf der Basis einer Flüssigkristallzelle, die ein derartiges flüssigkristallines Dielektrikum enthält.

Weiterhin Gegenstand der Erfindung ist ein Verfahren zur Herstellung der Verbindungen der Formel I nach Anspruch 1 dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,

oder daß man eine Verbindung, die sonst der Formel I entspricht, aber an Stelle von CN, F, Cl oder Br eine andere substituierbare Gruppe enthält, einer Substitution unterwirft,

oder daß man eine entsprechende Verbindung mit einer Abgangsgruppe $L^1$ mit der entsprechenden Verbindung mit einer Abgangsgruppe $L^2$ unter Abspaltung von $L^1 - L^2$ kondensiert,

oder daß man zur Herstellung von Dioxanderivaten der Formel I einen entsprechenden Aldehyd oder dessen Acetal mit einem entsprechenden Diol umsetzt,

oder daß man zur Herstellung von Pyrimidinderivaten der Formel I ein entsprechendes Amidin oder eines seiner funktionellen Derivate mit einem entsprechenden Malonsäurederivat umsetzt.

Vor- und nachstehend haben $R^1$, $R^2$, $R^3$, $R^4$, $R^5$, $R^6$, $R^7$, A, B, C, X und Y die angegebene Bedeutung, sofern nicht ausdrücklich etwas anderes vermerkt ist.

Der Einfachheit halber bedeuten im folgenden "Ph" einen Ring der Formel (1), worin $R^3$ CH bedeutet, "Py" einen Ring der Formel (1), worin $R^3$ N bedeutet, "Cy" einen Ring der Formel (2), worin $R^5$ $CH_2$ bedeutet, und "Dio" einen Ring der Formel (2), worin $R^5$ O bedeutet.

Die Verbindungen der Formel I umfassen dementsprechend beispielsweise Verbindungen der Teilformeln Ia bis Iz und Iaa bis Iqq:

| | |
|---|---|
| $R^1$-Ph-X-Cy-Y-Ph-$R^2$ | Ia |
| $R^1$-Ph-X-Dio-Y-Ph-$R^2$ | Ib |
| $R^1$-Cy-X-Cy-Y-Cy-$R^2$ | Ic |
| $R^1$-Cy-X-Dio-Y-Cy-$R^2$ | Id |
| $R^1$-Dio-X-Cy-Y-Dio-$R^2$ | Ie |
| $R^1$-Dio-X-Dio-Y-Dio-$R^2$ | If |
| $R^1$-Py-X-Cy-Y-Py-$R^2$ | Ig |
| $R^1$-Py-X-Dio-Y-Py-$R^2$ | Ih |
| $R^1$-Ph-X-Cy-Y-Cy-$R^2$ | Ii |
| $R^1$-Ph-X-Dio-Y-Cy-$R^2$ | Ij |
| $R^1$-Ph-X-Cy-Y-Dio-$R^2$ | Ik |
| $R^1$-Ph-X-Dio-Y-Dio-$R^2$ | Il |
| $R^1$-Ph-X-Cy-Y-Py-$R^2$ | Im |
| $R^1$-Ph-X-Dio-Y-Py-$R^2$ | In |
| $R^1$-Cy-X-Cy-Y-Dio-$R^2$ | Io |
| $R^1$-Cy-X-Dio-Y-Dio-$R^2$ | Ip |
| $R^1$-Cy-X-Cy-Y-Py-$R^2$ | Iq |
| $R^1$-Cy-X-Dio-Y-Py-$R^2$ | Ir |

$R^1$-Dio-X-Cy-Y-Py-$R^2$ Is

$R^1$-Dio-X-Dio-Y-Py-$R^2$ It

$R^1$-Cy-X-Ph-Ph-Y-Cy-$R^2$ Iu

$R^1$-Cy-X-Cy-Ph-Y-Cy-$R^2$ Iv

$R^1$-Cy-X-Cy-Cy-Y-Cy-$R^2$ Iw

$R^1$-Cy-X-Dio-Ph-Y-Cy-$R^2$ Ix

$R^1$-Cy-X-Dio-Cy-Y-Cy-$R^2$ Iy

$R^1$-Cy-X-Py-Ph-Y-Cy-$R^2$ Iz

$R^1-A^1-CH_2-[A^2]_n-CH_2-A^3-R^2$ Iaa

$R^1-A^1-O-[A^2]_n-O-A^3-R^2$ Ibb

$R^1-A^1-S-[A^2]_n-S-A^3-R^2$ Icc

$R^1-A^1-CH_2-[A^2]_n-O-A^3-R^2$ Idd

$R^1-A^1-CH_2-[A^2]_n-S-A^3-R^2$ Iee

$R^1-A^1-CH_2-[A^2]_n-CHR^7-A^3-R^2$ Iff

$R^1-A^1-CH_2-[A^2]_n-CH{=}CH-Q-A^3-R^2$ Igg

$R^1-A^1-CH_2-[A^2]_n-OCO-CH_2-A^3-R^2$ Ihh

$R^1-A^1-CH_2-[A^2]_n-COO-CH_2-A^3-R^2$ Iii

$R^1-A^1-CH_2-[A^2]_n-Q-CH_2-Q-A^3-R^2$ Ijj

$R^1-A^1-O-[A^2]_n-CHR^7-A^3-R^2$ Ikk

$R^1-A^1-O-[A^2]_n-CH{=}CH-Q-A^3-R^2$ Ill

$R^1-A^1-O-[A^2]_n-OCO-CH_2-A^3-R^2$ Imm

$R^1-A^1-O-[A^2]_n-OCO-CH_2-A^3-R^2$ Inn

$R^1-A^1-O-[A^2]_n-Q-CH_2-Q-A^3-R^2$ Ioo

$R^1-A^1-CH_2-[A^2]_n-CH_2-COO-A^3-R^2$ Ipp

$R^1-A^1-CH_2-[A^2]_n-CH_2-OCO-A^3-R^2$ Iqq

Darunter sind diejenigen der Teilformeln Ia, Ib, Ic, Id, Ii, Ij, Ik, Io, Ip, Iu, Iv, Iw, Ix und diejenigen der Teilformeln Iaa, Idd, Iff, Ihh, Imm, Inn, Ipp und Iqq besonders bevorzugt.

In den Verbindungen der vor- und nachstehenden Formeln bedeuten $R^1$ und $R^2$ vorzugsweise Alkyl oder Alkoxy, ferner Alkanoyloxy. Bevorzugt sind Verbindungen, in

denen einer der Reste $R_1$ und $R_2$ H, CN, F, Cl oder Br bedeutet. Weiterhin bevorzugt sind Verbindungen, die einen Ring der Formel (1) enthalten, welcher als Radikal $R^1$ oder $R^2$ einen Substituenten aus der Gruppe Alkoxy, Alkanoyloxy, H, F oder CN trägt.

In den Ringen der Formel (1) bedeutet $R^4$ vorzugsweise H, F, CN, $CH_3$ oder $OCH_3$; besonders bevorzugt sind neben nicht lateral substituierten Ringen diejenigen mit lateralem F oder CN.

In den Ringen der Formel (2) bedeutet $R^6$ vorzugsweise H, CN oder F.

In den Verbindungen der Formel I und Ia bis Iz bedeuten X und Y jeweils unabhängig voneinander vorzugsweise -O- oder $-CH_2-$.

In den Verbindungen der Formeln I und Iaa bis Iqq bedeutet $A^2$ vorzugsweise eine 1,4-Phenyl-, trans-1,4-Cyclohexylen- oder eine trans-1,3-Dioxan-2,5-diylgruppe.

In den Verbindungen der vor- und nachstehenden Formeln können die Alkyl-, Alkoxy- und Alkanoyloxyreste geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, Acetoxy, Propionyloxy, Butyryloxy, Pentanoyloxy, Hexanoyloxy oder Heptanoyloxy.

Verbindungen der Formeln I sowie Ia bis Iz und Iaa bis Iqq mit verzweigten Flügelgruppen $R_1$ bzw. $R_2$ können gelegentlich wegen einer besseren Löslichkeit in den üblichen flüssigkristallinen Basismaterialien

von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R_1$ und $R_2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Unter den Verbindungen der Formeln I sowie Ia bis Iz und Iaa bis Iqq sind diejenigen bevorzugt, in denen mindestens einer der darin enthaltenen Reste eine der angegebenen bevorzugten Bedeutungen hat.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe sind größtenteils bekannt. Alle lassen sich nach oder analog zu in der Literatur beschriebenen Standardmethoden darstellen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanringes einen Cyclohexenring oder Cyclohexanonring enthalten.

Die Reduktion erfolgt zweckmäßig durch katalytische Hydrierung bei Temperaturen zwischen etwa $0^{o}$ und etwa $200^{o}$ sowie Drucken zwischen etwa 1 und 200 bar in einem inerten Lösungsmittel, z.B. einem Alkohol wie Methanol, Ethanol oder Isopropanol, einem Ether wie Tetrahydrofuran oder Dioxan, einem Ester wie Ethylacetat, einer Carbonsäure wie Essigsäure oder einem Kohlenwasserstoff wie Cyclohexan.

Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form (z.B. Pt) eingesetzt werden können.

Verbindungen der Formel I sind weiterhin erhältlich, indem man eine Verbindung, die sonst der Formel I entspricht, aber an Stelle von CN, F, Cl oder Br eine andere substituierbare Gruppe enthält, einer Substitution unterwirft.

Zur Herstellung von Nitrilen der Formel I können entsprechende Chlor- oder Bromverbindungen der Formel I mit einem Cyanid umgesetzt werden, zweckmäßig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen etwa 20° und 200°.

Zur Herstellung von halogenhaltigen Verbindungen der Formel I können entsprechende Verbindungen der Formel I, die an Stelle von Halogen eine Aminogruppe enthalten, diazotiert und nachfolgend mit Kupfer(I)-bromid bzw. Kupfer(I)chlorid unter den Bedingungen einer Sandmeyer-Reaktion oder mit Tetrafluoroborat unter den Bedingungen einer Schiemann-Balz-Reaktion umgesetzt werden.

Die Diazotierung, die Sandmeyer-Reaktion und die Schiemann-Balz-Reaktion werden dabei in an sich bekannter Weise ausgeführt, wie sie zum Beispiel in Gattermann-Wieland, Die Praxis des organischen Chemikers, 40. Auflage (Walter de Gruyter & Co., Berlin 1961), Seiten 252 - 254 und in "Organic Reactions", Band 5 (1949), Seiten 193 - 228 beschrieben sind.

Zur Herstellung von Verbindungen der Formel I kann weiterhin eine entsprechende Verbindung mit einer Abgangsgruppe $L^1$ mit der entsprechenden Verbindung mit einer Abgangsgruppe $L^2$ unter Abspaltung von $L^1$-$L^2$ kondensiert werden.

Ether der Formel I sind beispielsweise durch Veretherung entsprechender Hydroxyverbindungen ($L^1$ = H), vorzugsweise Phenole, erhältlich, wobei die Hydroxyverbindung zweckmäßig zunächst in ein entsprechendes

Metallderivat ($L^1$ = Metall), z.B. durch Behandeln mit NaOH, KOH, $Na_2CO_3$ oder $K_2CO_3$ in das entsprechende Alkalimetallalkoholat oder Alkalimetallphenolat übergeführt wird. Dieses kann dann mit der entsprechenden Halogenverbindung ($L^2$ = Halogen) unter Abspaltung von Metallhalogenid ($L^1 - L^2$) umgesetzt werden, zweckmäßig in einem inerten Lösungsmittel wie Aceton, DMF oder Dimethylsulfoxid oder auch einen Überschuß an wässriger oder wässerig-alkoholischer NaOH oder KOH bei Temperaturen zwischen 20° und 100°. Bei Kondensationen unter Abspaltung von Halogenwasserstoff als Nebenprodukt $L^1$-$L^2$ kann zur Förderung der Reaktion eine organische oder anorganische Base zugesetzt werden (z.B. Pyridin oder Soda).

Einige der Verbindungen der Formel I sind beispielsweise durch Umsetzung von entsprechenden Grignard-Verbindungen oder analogen Lithiumkupraten mit der entsprechenden Halogen-, Alkylsulfonyloxy- oder Arylsulfonyloxy-Verbindung erhältlich ($L^1$-$L^2$ z.B. $MgHal_2$). Diese Umsetzungen werden in an sich bekannter Weise durchgeführt. Bei Verwendung von Grignard-Verbindungen wird zweckmäßigerweise Dilithiumtetrachlorokuprat oder Dilithiumtetrabromokuprat als Katalysator verwendet. Die Umsetzungen werden zweckmäßig in einem Ether, wie Diethylether, Tetrahydrofuran, Dioxan, Dimethoxyethan, Diethylenglykoldimethylether und dergleichen, ausgeführt. Temperatur und Druck können in weiten Grenzen gehalten werden. Im allgemeinen werden Atmosphärendruck und eine Temperatur zwischen etwa -80° und Raumtemperatur angewendet.

Dioxanderivate der Formel I werden zweckmäßig durch Reaktion eines entsprechenden Aldehyds oder eines seiner reaktionsfähigen Derivate mit einem entsprechenden 1,3-Diol oder einem seiner reaktionsfähigen Derivate hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°.

Pyrimidinderivate der Formel I werden zweckmäßig durch Reaktion eines entsprechenden Amidins oder eines seiner reaktionfähigen Derivate mit einem entsprechenden Malonsäurederivat hergestellt, vorzugsweise in Gegenwart eines Lösungsmittels, wie z.B. Methanol, Ethanol oder Isopropanol, und einer Base, und nachfolgender Halogeneinführung beispielsweise mit $POCl_3$, und Reduktion, vorzugsweise durch katalytische Hydrierung.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexylester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel II charakterisieren.

Die erfindungsgemäßen Dielektrika enthalten etwa 0,1 bis 100, vorzugsweise 10 bis 100 %, von Verbindungen der Formel I, wobei bei Abwesenheit anderer Verbindungen mindestens zwei Verbindungen der Formel I vorliegen.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyldodecyl-ammonium-4-hexyloxybenzoat, Tetrabutyl-ammonium-tetraphenylboranat oder Komplexsalze von Kronenethern (vgl. z.B. I. Haller et al., Mol.Cryst.Liq.Cryst. Band 24, Seiten 249 - 258 (1973)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. Vor- und nachstehend bedeuten Prozentangaben Gewichtsprozent; alle Temperaturen sind in Grad Celsius angegeben.

Beispiel 1

a) Zu einer auf -10° gekühlten Suspension von 27 g $AlCl_3$ in 400 ml $CS_2$ wird unter Rühren eine Lösung von 20 g Cyclohexan-trans-1,4-dicarbonsäuredichlorid in 75 ml $CS_2$ getropft. Anschließend wird unter Rühren eine Lösung von 30 g n-Propylbenzol in 50 ml $CS_2$ so zugetropft, daß die Temperatur nicht über 0° steigt. Nach Beendigung der HCl-Entwicklung läßt man auf Raumtemperatur erwärmen und gießt das Reaktionsgemisch unter Rühren auf eine Mischung von 200 ml konzentrierter Salzsäure und 1 kg zerstoßenem Eis. Die organische Phase wird abgetrennt, über $CaCl_2$ getrocknet und eingedampft; aus dem Rückstand werden durch Umkristallisation aus Petrolether (Siedebereich 60 - 80°) 22 g trans-1,4-bis-(4-n-Propylbenzoyl)-cyclohexan erhalten.

b) Eine Mischung von 25 g amalgamiertem Zink, 20 ml Wasser, 60 ml 24 %iger Salzsäure, 50 ml Toluol und 22 g trans-1,4-bis-(4-n-Propylbenzoyl)-cyclohexan wird 30 Stunden lang zum Sieden erhitzt, wobei nach je 6 Stunden je 10 ml konzentrierte Salzsäure zugefügt werden. Nach dem Abkühlen wird die organische Phase abgetrennt und die wässrige Phase noch zweimal mit je 50 ml Toluol ausgeschüttelt. Die vereinigten organischen Phasen werden über $CaCl_2$ getrocknet und eingedampft; das zurückbleibende trans-1,4-bis-(4-n-Propylbenzyl)-cyclohexan wird aus Ethanol umkristallisiert.

Analog werden hergestellt:

trans-1-(4-n-Propylbenzyl)-4-(4-n-butylbenzyl)-cyclohexan

trans-1-(4-n-Propylbenzyl)-4-(4-n-pentylbenzyl)-cyclohexan

trans-1-(4-n-Propylbenzyl)-4-(4-n-hexylbenzyl)-cyclohexan

trans-1-(4-n-Propylbenzyl)-4-(4-n-heptylbenzyl)-cyclohexan

trans-1-(4-n-Butylbenzyl)-4-(4-n-propylbenzyl)-cyclohexan

trans-1-(4-n-Butylbenzyl)-4-(4-n-pentylbenzyl)-cyclohexan

trans-1-(4-n-Butylbenzyl)-4-(4-n-hexylbenzyl)-cyclohexan

trans-1-(4-n-Butylbenzyl)-4-(4-n-heptylbenzyl)-cyclohexan

trans-1-(4-n-Pentylbenzyl)-4-(4-n-propylbenzyl)-cyclohexan

trans-1-(4-n-Pentylbenzyl)-4-(4-n-butylbenzyl)-cyclohexan

trans-1-(4-n-Pentylbenzyl)-4-(4-n-hexylbenzyl)-cyclohexan

trans-1-(4-n-Pentylbenzyl)-4-(4-n-heptylbenzyl)-cyclohexan

trans-1-(4-n-Hexylbenzyl)-4-(4-n-propylbenzyl)-cyclohexan

trans-1-(4-n-Hexylbenzyl)-4-(4-n-butylbenzyl)-cyclohexan

trans-1-(4-n-Hexylbenzyl)-4-(4-n-pentylbenzyl)-cyclohexan

trans-1-(4-n-Heptylbenzyl)-4-(4-n-propylbenzyl)-cyclohexan

trans-1,4-bis-(4-n-Butylbenzyl)-cyclohexan

trans-1,4-bis-(4-n-Pentylbenzyl)-cyclohexan

trans-1,4-bis-(4-n-Hexylbenzyl)-cyclohexan

trans-1-(4-n-Heptylbenzyl)-4-(4-n-butylbenzyl)-cyclohexan
trasn-1-(4-n-Heptylbenzyl)-4-(4-n-pentylbenzyl)-cyclohexan

Beispiel 2

Eine Lösung von 19 g 4-Cyanophenylacetaldehyd und 24,1 g 4-(4-n-Pentylphenyl)-1,1-bis-(hydroxymethyl)-butan in 150 ml Toluol wird in Gegenwart von 120 mg p-Toluolsulfonsäure 8 Stunden am Wasserabscheider zum Sieden erhitzt. Nach Abkühlen wird das Reaktionsgemisch mit 20 ml 5 %iger wäßriger Natriumhydrogencarbonatlösung gewaschen, die organische Phase über $MgSO_4$ getrocknet und eingedampft. Das zurückbleibende 2-(4-Cyanobenzyl)-5-(3-p-n-pentylphenylpropyl)-1,3-dioxan wird aus Methanol umkristallisiert.

Analog werden hergestellt:

2-(4-Cyanobenzyl)-5-(3-p-methylphenylpropyl)-1,3-dioxan
2-(4-Cyanobenzyl)-5-(3-p-ethylphenylpropyl)-1,3-dioxan
2-(4-Cyanobenzyl)-5-(3-p-n-propylphenylpropyl)-1,3-dioxan
2-(4-Cyanobenzyl)-5-(3-p-n-butylphenylpropyl)-1,3-dioxan
2-(4-Cyanobenzyl)-5-(3-p-n-hexylphenylpropyl)-1,3-dioxan
2-(4-Cyanobenzyl)-5-(3-p-n-heptylphenylpropyl)-1,3-dioxan
2-(4-Cyanobenzyl)-5-(3-p-n-octylphenylpropyl)-1,3-dioxan
2(4-Cyanobenzyl)-5-(3-p-methoxyphenylpropyl)-1,3-dioxan
2(4-Cyanobenzyl)-5-(3-p-ethoxyphenylpropyl)-1,3-dioxan

2(4-Cyanobenzyl)-5-(3-p-n-propyloxyphenylpropyl)-1,3-dioxan
2(4-Cyanobenzyl)-5-(3-p-n-butoxyphenylpropyl)-1,3-dioxan
2(4-Cyanobenzyl)-5-(3-p-n-pentoxyphenylpropyl)-1,3-dioxan
2(4-Cyanobenzyl)-5-(3-p-n-hexoxyphenylpropyl)-1,3-dioxan
2(4-Cyanobenzyl)-5-(3-p-n-heptoxyphenylpropyl)-1,3-dioxan
2(4-Cyanobenzyl)-5-(3-p-n-octoxyphenylpropyl)-1,3-dioxan
5(4-Cyanobenzyl)-2-(3-p-n-propyloxyphenylpropyl)-1,3-dioxan
5(4-Cyanobenzyl)-2-(3-p-n-propyloxyphenylmethyl)-1,3-dioxan
5(4-Cyanobenzyl)-2-(3-p-n-pentylphenylmethyl)-1,3-dioxan
5(4-Cyanobenzyl)-2-(3-p-n-pentylphenylpropyl)-1,3-dioxan
2-(4-Cyanobenzyl)-5-(3-p-methylphenylmethyl)-1,3-dioxan
2-(4-Cyanobenzyl)-5-(3-p-ethylphenylmethyl)-1,3-dioxan
2-(4-Cyanobenzyl)-5-(3-p-n-propylphenylmethyl)-1,3-dioxan
2-(4-Cyanobenzyl)-5-(3-p-n-butylphenylmethyl)-1,3-dioxan
2-(4-Cyanobenzyl)-5-(3-p-n-pentylphenylmethyl)-1,3-dioxan
2-(4-Cyanobenzyl)-5-(3-p-n-hexylphenylmethyl)-1,3-dioxan
2-(4-Cyanobenzyl)-5-(3-p-n-heptylphenylmethyl)-1,3-dioxan
2-(4-Cyanobenzyl)-5-(3-p-n-octylphenylmethyl)-1,3-dioxan
2-(4-Cyanobenzyl)-5-phenylmethyl-1,3-dioxan
2-(4-Cyanobenzyl)-5-phenylpropyl-1,3-dioxan
5-(4-Cyanobenzyl)-2-phenylmethyl-1,3-dioxan
5-(4-Cyanobenzyl)-2-phenylpropyl-1,3-dioxan

Beispiel 3

a) 22,4 g einer 50 Gew.%igen Lithiumsuspension in Cyclohexan und 3 Tropfen 1,2-Dibromethan werden unter Argonatmosphäre auf $-10^{o}$ gekühlt. Unter Rühren werden hierzu 103 g p-n-Propyl-ω-bromstyrol in 150 ml Ether so zugetropft, daß die Temperatur $10^{o}$ nicht übersteigt. Ein Niederschlag beginnt sich zu bilden. Das Gemisch wird noch 30 Minuten bei Raumtemperatur gerührt und durch Glaswolle filtriert.

b) Ein Drittel dieser Lösung wird unter Argon bei -78° unter Rühren zu 12,6 g Cu(I)-jodid in 100 ml Ether während einer Stunde getropft. Man läßt das Gemisch auf -35° erwärmen und tropft 44 ml Hexamethylphosphorsäuretriamid zu, wobei sich ein weißer Niederschlag bildet. Zu diesem Gemisch werden bei -35° 25,6 g trans-1-Jodmethyl-4-(trans-4-n-pentylcyclohexylmethyl)-cyclohexan unter Rühren zugetropft. Man läßt auf 0° erwärmen und gießt in 400 ml gesättigte Ammoniumchloridlösung. Nach Filtration wird die wäßrige Phase dreimal mit 100 ml Petrolether gewaschen. Die vereinigten organischen Phasen werden mit gesättigter Ammoniumchloridlösung und mit gesättigter Natriumchloridlösung gewaschen, über Magnesiumsulfat getrocknet und eingedampft. Das zurückbleibende trans-1-(trans-4-n-Pentylcyclohexylmethyl)-4-(3-p-n-propylphenyl-2-propenyl)-cyclohexan wird aus Petrolether umkristallisiert.

Beispiel 4

Eine Lösung von 3,2 g trans-4-n-Propylcyclohexyl-4'-trans-4-n-propylcyclohexyl-biphenylyl-4-keton [F. 133°; erhältlich durch Umsetzung von 4-(trans-4-n-Propylcylclohexylmethyl)-biphenyl (F. 103°) mit trans-4-n-Propylcyclohexylcarbonsäurechlorid unter Friedel-Crafts-Bedingungen (Dichlormethan/Aluminiumchlorid). 4-(trans-4-n-Propylcyclohexylmethyl)-biphenyl ist erhältlich durch Reduktion von Biphenylyl-4-trans-4-n-propylcyclohexylketon, welches durch Friedel-Crafts-Acylierung von Biphenyl mit trans-4-n-Propylcyclohexancarbonsäurechlorid erhältlich ist], 1,6 g Kaliumhydroxid und 1,1 g Hydrazinhydrat in 10 ml Diethylenglykol wird im Lauf von 3 Stunden von 100° auf 195° erwärmt.

Nach dem Erkalten wird das Reaktionsgemisch mit 15 ml Wasser versetzt und mit 10 ml Dichlormethan extrahiert. Aus dem Extrakt werden das Lösungsmittel und mit diesem azeotrop die Restfeuchtigkeit abdestilliert und das zurückbleibende 4,4'-Bis(trans-4-n-propylcyclohexylmethyl)-biphenyl aus Ethanol umkristallisiert; Ausbeute 1,8 g, F. 87°, K. 95°.

Analog werden hergestellt:

trans-1,4-bis-(trans-4-n-Propylcyclohexylmethyl)-cyclohexan
trans-1,4-bis-(trans-4-n-Butylcyclohexylmethyl)-cyclohexan
trans-1,4-bis-(trans-4-n-Pentylcyclohexylmethyl)-cyclohexan
trans-1,4-bis-(trans-4-n-Hexylcyclohexylmethyl)-cyclohexan
trans-1,4-bis-(trans-4-n-Heptylcyclohexylmethyl)-cyclohexan
trans-1-(trans-4-n-Propylcyclohexylmethyl)-4-(trans-4-n-butylcyclohexylmethyl)-cyclohexan
trans-1-(trans-4-n-Propylcyclohexylmethyl)-4-(trans-4-n-pentylcyclohexylmethyl)-cyclohexan
trans-1-(trans-4-n-Propylcyclohexylmethyl)-4-(trans-4-n-hexylcyclohexylmethyl)-cyclohexan
trans-1-(trans-4-n-Propylcyclohexylmethyl)-4-(trans-4-n-heptylcyclohexylmethyl)-cyclohexan
trans-1-(trans-4-n-Butylcyclohexylmethyl)-4-(trans-4-n-propylcyclohexylmethyl)-cyclohexan
trans-1-(trans-4-n-Butylcyclohexylmethyl)-4-(trans-4-n-pentylcyclohexylmethyl)-cyclohexan
trans-1-(trans-4-n-Butylcyclohexylmethyl)-4-(trans-4-n-hexylcyclohexylmethyl)-cyclohexan
trans-1-(trans-4-n-Butylcyclohexylmethyl)-4-(trans-4-n-heptylcyclohexylmethyl)-cyclohexan
trans-1-(trans-4-n-Pentylcyclohexylmethyl)-4-(trans-4-n-propylcyclohexylmethyl)-cyclohexan

trans-1-(trans-4-n-Pentylcyclohexylmethyl)-4-(trans-4-n-butylcyclohexylmethyl)-cyclohexan
trans-1-(trans-4-n-Pentylcyclohexylmethyl)-4-(trans-4-n-hexylcyclohexylmethyl)-cyclohexan
trans-1-(trans-4-n-Hexylcyclohexylmethyl)-4-(trans-4-n-propylcyclohexylmethyl)-cyclohexan
trans-1-(trans-4-n-Hexylcyclohexylmethyl)-4-(trans-4-n-butylcyclohexylmethyl)-cyclohexan
trans-1-(trans-4-n-Hexylcyclohexylmethyl)-4-(trans-4-n-pentylcyclohexylmethyl)-cyclohexan
trans-1-(trans-4-n-Propylcyclohexylmethyl)-4-(p-n-propyl-benzyl)-cyclohexan
trans-1-(trans-4-n-Propylcyclohexylmethyl)-4-(benzyl)-cyclohexan
trans-1-(trans-4-n-Propylcyclohexylmethyl)-4-(p-fluorbenzyl)-cyclohexan
trans-1-(trans-4-n-Propylcyclohexylmethyl)-4-(p-cyano-benzyl)-cyclohexan
trans-1-(trans-4-n-Propylcyclohexylmethyl)-4-(p-n-butyl-benzyl)-cyclohexan
trans-1-(trans-4-n-Propylcyclohexylmethyl)-4-pentyl-cyclohexan
trans-1-(trans-4-n-Propylcyclohexylmethyl)-4-hexylcyclohexan
trans-1-(trans-4-n-Propylcyclohexylmethyl)-4-heptyl-cyclohexan
trans-1-(trans-4-n-Pentylcyclohexylmethyl)-4-propyl-cyclohexan
trans-1-(trans-4-n-Pentylcyclohexylmethyl)-4-butyl-cyclohexan
trans-1-(trans-4-n-Pentylcyclohexylmethyl)-4-pentyl-cyclohexan
trans-1-(trans-4-n-Pentylcyclohexylmethyl)-4-heptyl-cyclohexan

trans-1-(trans-4-n-Heptylcyclohexylmethyl)-4-propyl-cyclohexan
trans-1-(trans-4-n-Heptylcyclohexylmethyl)-4-butyl-cyclohexan
trans-1-(trans-4-n-Heptylcyclohexylmethyl)-4-pentyl-cyclohexan
trans-1-(trans-4-n-Heptylcyclohexylmethyl)-4-hexylcyclohexan
trans-1-(trans-4-n-Heptylcyclohexylmethyl)-4-heptyl-cyclohexan
trans-trans-1,4'-bis-(trans-4-n-Propylcyclohexylmethyl)-cyclohexylcyclohexan
trans-trans-1,4'-bis-(trans-4-n-Butylcyclohexylmethyl)-cyclohexylcyclohexan
trans-trans-1,4'-bis-(trans-4-n-Pentylcyclohexylmethyl)-cyclohexylcyclohexan
trans-trans-1,4'-bis-(trans-4-n-Hexylcyclohexylmethyl)-cyclohexylcyclohexan
trans-trans-1,4'-bis-(trans-4-n-Heptylcyclohexylmethyl)-cyclohexylcyclohexan
trans-trans-1,4'-bis-(trans-4-n-Octylcyclohexylmethyl)-cyclohexylcyclohexan
trans-trans-1-(trans-4-n-Propylcyclohexylmethyl)-4-(trans-4-n-butylcyclohexylmethyl)-cyclohexylcyclohexan
trans-trans-1-(trans-4-n-Propylcyclohexylmethyl)-4-(trans-4-n-pentylcyclohexylmethyl)-cyclohexylcyclohexan
trans-trans-1-(trans-4-n-Propylcyclohexylmethyl)-4-(trans-4-n-hexylcyclohexylmethyl)-cyclohexylcyclohexan
trans-trans-1-(trans-4-n-Propylcyclohexylmethyl)-4-(trans-4-n-heptylcyclohexylmethyl)-cyclohexylcyclohexan
trans-trans-1-(trans-4-n-Butylcyclohexylmethyl)-4-(trans-4-n-propylcyclohexylmethyl)-cyclohexylcyclohexan
trans-trans-1-(trans-4-n-Butylcyclohexylmethyl)-4-(trans-4-n-pentylcyclohexylmethyl)-cyclohexylcyclohexan

trans-trans-1-(trans-4-n-Butylcyclohexylmethyl)-4-(trans-4-n-hexylcyclohexylmethyl)-cyclohexylcyclohexan
trans-trans-1-(trans-4-n-Butylcyclohexylmethyl)-4-(trans-4-n-heptylcyclohexylmethyl)-cyclohexylcyclohexan
trans-trans-1-(trans-4-n-Butylcyclohexylmethyl)-4-(trans-4-n-cyanocyclohexylmethyl)-cyclohexylcyclohexan
trans-trans-1-(trans-4-n-Pentylcyclohexylmethyl)-4-cyclohexylmethyl-cyclohexylcyclohexan
trans-trans-1-(trans-4-n-Pentylcyclohexylpropyl)-4-cyclohexylmethyl-cyclohexylcyclohexan

Beispiel 5

Ein flüssigkristallines Dielektrikum aus

20 % p-trans-4-n-Propylcyclohexyl-benzonitril
30 % p-trans-4-n-Pentylcyclohexyl-benzonitril
10 % p-trans-4-n-Heptylcyclohexyl-benzonitril
12 % 4-Cyan-4'-(trans-4-n-pentylcyclohexyl)-biphenyl
18 % 4,4'-Bis(trans-4-n-propylcyclohexylmethyl)-biphenyl
10 % trans-4-n-Butylcyclohexancarbonsäure-(p-trans-4-n-propylcyclohexylester)

hat einen Klärpunkt von 82°, eine dielektrische Anisotropie von $\Delta E = +8{,}0$, eine optische Anisotropie von $\Delta n = 0{,}13$ und eine Viskosität von 35 $mm^2/s$ bei 20° und ist gut für Flüssigkristall-Anzeigeelemente auf der Basis der verdrillten nematischen Zelle geeignet.

Merck Patent Gesellschaft
mit beschänkter Haftung
D a r m s t a d t

Patentansprüche

1. Verbindungen der Formel I

$R^1$- $A^1$ -X-$[A^2]_n$-Y- $A^3$ - $R^2$

worin

$A^1$, $A^2$ und $A^3$ jeweils unabhängig voneinander einen Ring der Formel (1) oder (2),

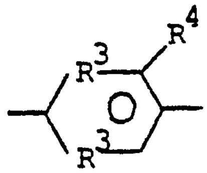

(1)

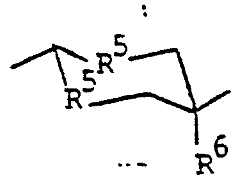

(2)

n 1 oder 2,

X und Y jeweils unabhängig voneinander -O-, -S-, $-CH_2-$, einer davon auch $-CHR^7-$, -CH=CH-Q, $-OCO-CH_2-$, $-COO-CH_2-$ oder $-Q-CH_2-Q-$,

$R^1$ und $R^2$ Alkyl, Alkoxy oder Alkanoyloxy mit jeweils bis zu 8 C-Atomen, einer davon auch H, CN, F, Cl oder Br,

Q O, S oder $CH_2$,

$R^3$ CH oder N,

$R^4$ H, CN, F, Cl, Br, $CH_3$ oder $OCH_3$,

$R^5$ $CH_2$ oder O,

$R^6$ H, CN, F, Cl oder Br und

$R^7$ CN, F, Cl oder Br bedeutet.

2. Verfahren zur Herstellung der Verbindungen der Formel I nach Anspruch 1 dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,

oder daß man eine Verbindung, die sonst der Formel I entspricht, aber an Stelle von CN, F, Cl oder Br eine andere substituierbare Gruppe enthält, einer Substitution unterwirft,

oder daß man eine entsprechende Verbindung mit einer Abgangsgruppe $L^1$ mit der entsprechenden Verbindung mit einer Abgangsgruppe $L^2$ unter Abspaltung von $L^1 - L^2$ kondensiert,

oder daß man zur Herstellung von Dioxanderivaten der Formel I einen entsprechenden Aldehyd oder dessen Acetal mit einem entsprechenden Diol umsetzt,

oder daß man zur Herstellung von Pyrimidinderivaten der Formel I ein entsprechendes Amidin oder eines seiner funktionellen Derivate mit einem entsprechenden Malonsäurederivat umsetzt.

3. Verwendung der Verbindungen der Formel I nach Anspruch 1 als Komponenten flüssigkristalliner Dielektrika.

4. Flüssigkristallmischung, dadurch gekennzeichnet, daß sie als Komponente mindestens eine Verbindung der Formel I nach Anspruch 1 enthält

5. Elektrooptisches Anzeigeelement, dadurch gekennzeichnet, daß es eine Flüssigkristallmischung nach Anspruch 4 enthält.

Europäisches Patentamt

# EUROPÄISCHER RECHERCHENBERICHT

0131837

Nummer der Anmeldung

EP 84 10 7668

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl. 3) |
|---|---|---|---|
| X | GB-A-2 081 301 (NIPPON STEEL CHEMICAL CO.)<br>* Patentansprüche *<br>----- | 1 | C 07 C 13/28<br>C 07 C 25/18<br>C 07 C 121/00<br>C 07 D 319/06<br>C 09 K 19/30<br>C 09 K 19/34 |
| | | | RECHERCHIERTE SACHGEBIETE (Int. Cl. 3) |
| | | | C 07 C 13/00<br>C 07 C 25/00<br>C 07 C 121/00<br>C 07 D 319/00<br>C 09 K 19/00 |

Der vorliegende Recherchenbericht wurde für alle Patentansprüche erstellt.

| Recherchenort | Abschlußdatum der Recherche | Prüfer |
|---|---|---|
| DEN HAAG | 08-10-1984 | VAN GEYT J.J.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82